Europäisches Patentamt

European Patent Office

Office européen des brevets

(11) Publication number: **0 163 141 B1**

# EUROPEAN PATENT SPECIFICATION

(45) Date of publication of patent specification: **28.08.91**  (51) Int. Cl.⁵: **C12P 21/08**

(21) Application number: **85105129.2**

(22) Date of filing: **26.04.85**

(54) Monoclonal anti-human IgG antibody and process for preparing the same.

(30) Priority: **27.04.84 JP 87190/84**

(43) Date of publication of application:
**04.12.85 Bulletin 85/49**

(45) Publication of the grant of the patent:
**28.08.91 Bulletin 91/35**

(84) Designated Contracting States:
**BE CH DE FR IT LI NL SE**

(56) References cited:
**EP-A- 0 088 974**
**US-A- 4 468 346**

**HYBRIDOMA, vol. 1, no. 3, 1982, pages
257-273, Mary Ann Liebert, Inc., Publishers,
Chicago, US; T.A. SPRINGER et al.:
"Monoclonal antibodies specific for rat IgG1,
IgG2a, IgG2b subclasses, and kappa chain
monotypic and allotypic determinants:
reagents for use with rat monoclonal anti-
bodies"**

**NATURE, vol. 277, 11th January 1979, pages
131-133, Macmillan Journals Ltd, London,
GB; G. GALFRE et al.: "Rat x rat hybrid
myelomas and a monoclonal anti-Fd portion
of mouse IgG"**

(73) Proprietor: **SHIONOGI & CO., LTD.**
**1-8, Doshomachi 3-chome Chuo-ku**
**Osaka 541(JP)**

(72) Inventor: **Mikawa, Haruki**
**6-16, Oharanokamizatokatsuyama-cho**
**Nishigyo-ku**
**Kyoto-shi Kyoto(JP)**
Inventor: **Hosoi, Susumu Kotohait-**
**sufushimiinari C-525**
**1-1, Fukakusashimogawara-cho**
**Fushimi-ku Kyoto-shi Kyoto(JP)**

(74) Representative: **Vossius & Partner**
**Siebertstrasse 4 P.O. Box 86 07 67**
**W-8000 München 86(DE)**

## Description

In allergic diseases caused by antigen-antibody reactions, the research and identification of the allergen made possible diagnosis and classification of the diseases and working out of a therapy. The monoclonal antibody of the present invention is a useful diagnostic reagent for identifying an allergen causing allergic diseases such as extrinsic bronchial asthma, atopic dermatitis and immune complex disease. Furthermore, it is useful for elucidating the mechanism of the genesis of allergic diseases.

In the last few decades it was found that Immunoglobulin G (IgG)levels against an allergen in human serum are a significant parameter for allergic diseases (J.W. Younginger et al., J.Clin. Invest. 52, 1268 (1973); A.K. Sobotka et al., J. Immunol. 117, 84 (1976)). Recently, a radioallergosorbent test (hereinafter referred to as RAST) has been utilized for determination of levels of specific IgE against an allergen in human serum (N.F. Adkinson, Jr., American Society for Microbiology, Washington, D.C., p. 590-602 (1976)). RAST, however, can not be used for the measurement of human allergen-specific IgG, except in some cases which are disclosed in a few reports (Shimizu et al., J. Immunol. Methods, 19, 317 (1978)). It is speculated that RAST can not be used for this purpose since the binding level of a non-specific antibody in normal serum is higher than that of a specific antibody. Hamilton et al. succeeded in reducing the non-specific binding level. They used $^{125}$I-protein A, separated from Staphylococcus aureus, instead of a rabbit anti-human IgG antibody (J. Immunol. 122, 1073 (1979)). The protein A separated from Staphylococcus aureus binds specifically to the Fc site of the human IgG subclasses 1, 2 and 4 but not to human IgG$_3$. Though the protein A binds to IgA$_1$ and IgA$_2$, which are subclasses of Immunoglobulin A (IgA), it is likely that there is no special relation between these IgA subclasses and protein A (Buruda et al., J. Immunol., Vol 123, 1457 (1979)). Johanson and Inganas have noted that Immunoglobulin E (hereinafter referred to as IgE) separated from the serum containing polyclonal IgE has a high affinity to the protein A. In contrast to IgG, the binding site of IgE is not the Fc fragment (Immunological Rev. 41, 248 (1978)).

Therefore, the present invention relates to a novel method for measurement of human allergen-specific IgG. The monoclonal anti-human IgG antibody according to the present invention binds with an equal affinity to specific antigenic determinants of human IgG. A large amount of this monoclonal antibody can be produced in the abdominal cavity of a mouse. Allergen-specific human IgG levels can be determined using this monoclonal antibody which reliably recognizes allergen-specific human IgG.

The monoclonal anti-human IgG antibody provided by the present invention is characterized as follows:
(1) Recognition of the CH$_2$-domain of human IgG,
(2) High affinity to human IgG and the IgG subclasses IgG$_1$, IgG$_2$, IgG$_3$ and IgG$_4$
(3) Better binding to the allergen-specific IgG than to the immobilized non-specific IgG,
(4) Nearly no affinity to free human IgG in the form of a solution

Furthermore, this monoclonal antibody is useful for measuring allergen-specific IgG and immune complexes by assays as RAST or ELISA (enzyme-linked immunosorbent assay). The antibody can also be used for elucidating the mechanism of the genesis of allergic diseases. Said monoclonal antibody has little non-specific affinity in normal serum. It can also be applied in measuring the allergen-specific IgG using the commercially available allergen disk or allergen-fixed carrier (for example, tube coating, beads and the like) prepared for measuring IgE.

Figure 1 shows a chromatography of the $^{125}$I-labeled monoclonal antibody HG2-25, in which the x-axis and the y-axis indicate the fraction number and the number of counts (%), respectively.

Figures 2 and 3 show Scatchard plots of the monoclonal antibody HG2-25, in which F and B are the molarity of HG2-25 not binding to human IgG and of the human IgG/HG2-25 complex, respectively.

The preparation of a monoclonal antibody against a human immunoglobulin as IgG comprises the following steps:
(1) Preparation of Antibody-Producing Spleen Cells
An animal as a mouse or a rat is immunized by administering intraperitoneally immunoglobulin from human serum in combination with complete Freund's adjuvant. After about 3 weeks for further stimulation an additional amount of immunoglobulin from human serum is administered interperitoneally together with an alum adjuvant. 3 days later the spleen is removed and antibody-producing cells for cell fusion are isolated.
(2) Preparation of Myeloma Cells
A myeloma cell line which is hypoxanthine-guanine-phosphoribosyl-transferase deficient, for example, myeloma cell P3-NS-1/1-Ag-4-1 (hereinafter referred to as NS-1/Ag) of the mouse strain BALB/C (Proc. Natl. Acad. Sci., USA, 76, 4061 (1979)), is cultivated in a medium, for example, RPMI-1640 medium containing 15% fetal calf serum (FCS). NS-1/Ag does not grow in a hypoxanthine-aminopterin-thymidine-medium (HAT medium).

(3) Preparation of Hybridoma Cells

The spleen cells prepared according to (1) and cells of the myeloma NS-1/Ag prepared according to (2) are fused in the presence of a polyethylene glycol (PEG) according to the method of Oi and Herzenberg (Selected Methods in Cellular Immunology, 351-372 (1980), San Francisco, W.H. Freeman and Co.) The resulting hybrid cells are suspended in HAT medium and inoculated into wells of tissue culture plates. For supporting the growth of the hybrid cells a small amount of thymocyte or peritoneal percolating cells of the mouse strain BALB/C is added as feeder cells. 1, 2, 3, 5, 8 and 11 days after the beginning of the incubation, half the amount of the HAT medium is replaced by fresh HAT medium, and after 14, 15 and 16 days by a medium containing only hypoxanthine and thymidine (HT medium).

(4) Identification of Hybridomas Producing an Anti-Human Immunoglobulin Antibody

    1) Passive Hemagglutination Assay with Sheep Erythrocytes Sensitized with Human Immunoglobulin Sheep erythrocytes are washed with chromium(III) chloride and sensitized with human IgG according to the chromium chloride method of E.R. Gold and H.H. Fudenberg (J. Immunol., 99, 859 (1976)). In the same way sheep erythrocytes are produced which are sensitized with the Fc and F(ab')$_2$ fragment of human immunoglobulin and human myeloma protein. The antibody activity in the supernatant of the culture medium is examined with these sensitized erythrocytes in passive hemagglutination assay.

2) ELISA

Human immunoglobulin is fixed on a polystyrene plate with 0.05 M carbonate buffer (pH 9.1). After the remaining binding sites of the polystyrene plate were saturated with bovine serum albumin the plate was incubated with an aliquot of the medium in which the hybridomas were cultivated. After washing the plate, it is incubated with an anti-mouse immunoglobulin antibody which is conjugated with peroxidase and which shows no cross reactivity to human immunoglobulin. The obtained complex adhering to the plate is then incubated with the substrate hydrogen peroxide. the reaction is stopped with sodium azide, and the absorbance is measured.

(5) Cloning and Selection of a Hybridoma

Hybridomas showing antibody activity in the previous assay (4) are cloned according to the known limited dilution method. The antibody activity in each medium in which a hybridoma was cultivated is determined as described under (4) above and a clone displaying high antibody activity is selected.

(6) Preparation of a Monoclonal Anti-Human Immunoglobulin (e.g. IgG)Antibody

The clone selected in (5) is incubated in a suitable medium, for example RPMI-1640 medium containing 15% FCS until the number of the cells reaches a maximum density. Alternatively, the clone selected can be transplanted in the abdominal cavity of BALB/C mice preliminarily treated with pristane. After 3 weeks the accumulated ascites is isolated.

(7) Isolation and Purification of the Monoclonal Antibody

The monoclonal antibody can easily be isolated from the ascites or the supernatant of a hybridoma culture and purified by the method well known in this field , for example, affinity chromatography with the protein A Sepharose.


Example 1

(1) Preparation of Anti-Human IgG Antibody-producing Hybridomas

For primary immunization, 200 $\mu$g of IgG from human serum (100 $\mu$l of physiological saline) together with 100 $\mu$l of a complete Freund's adjuvant were administered intraperitoneally to BALB/C mice (age 8-10 weeks). After 21 days, 20 $\mu$g of the human IgG were administered intraperitoneally to these mice as an alum sediment. Three days after this second immunization, the spleen was removed, the spleen cells were isolated according to known methods and suspended in RPMI medium. 10 ml RPMI medium containing $10^8$ spleen cells were mixed with a suspension of $5 \times 10^7$ NS-1 myeloma cells in 10 ml RPMI medium, and the mixture was centrifuged at 450xg for 10 minutes. The supernatant was discarded. Then 1 ml RPMI medium (RPMI-1640) containing 50% PEG 4000 (Merck & Co.) was slowly added to the cell pellet while stirring. The solution was stirred for an additional minute and 1 ml of said RPMI solution was added during the next minute. After addition of a further ml as described before, 7 ml of the same solution were added over about 3 minutes. Finally, the supernatant was removed by centrifugation at 400xg for 10 minutes. The cells obtained were suspended in 20 ml of RPMI-1640 medium containing 15% fetal calf serum (FCS). Each well of two tissue culture plates (Corning, 96-well) was inoculated with 100 $\mu$l cell suspension. The plates were incubated at 37°C in the presence of 7% carbonic dioxide gas. After 1, 2, 3, 5 and 8 days half the amount of the medium of each well was replaced by fresh HAT medium (RPMI-1640 medium, supplemented with 10% FCS, $4 \times 10^{-7}$M aminopterin, $1.6 \times 10^{-5}$M thymidine and $1 \times 10^{-4}$M hypoxanthine). At the 11th, 13th and 14th day half the amount of the medium was replaced by HT medium (RPMI medium containing 10% FCS, $1.6 \times 10^{-5}$ M thymidine and $1 \times 10^{-4}$ M hypoxanthine).

16 days after the fusing procedure hybridoma colonies were detected in each well. The resulting hybridomas were incubated in RPMI-1640 medium containing 15% FCS and the supernatant was examined for specific antibodies produced by these hybridomas.

5 $\mu$l of the supernatant solution to be examined were inoculated into each well of U-bottom tissue culture plates. 50 $\mu$l of a phosphate-buffered saline (PBS) containing 10% FCS and 50 $\mu$l of a 1% suspension of sheep erythrocytes sensitized with human IgG were added and the suspension was stirred moderately.

After 2 hours incubation at room temperature it was determined if hemagglutination had occurred. The results are shown in Table I.

## Table I

| Total number of wells | Number of wells in which a colony was observed | Number of anti-body-producing wells |
|---|---|---|
| 192 | 192 | 72 |

### (2) Cloning of Anti-Human IgG Antibody-Producing Cell Lines

Anti-human IgG antibody-producing hybridomas isolated as described in (1) were suspended in RPMI-1640 containing 15% FCS. The concentration was 5 cells per ml. 40 wells of the tissue culture plates (Corning, 96-well) were each inoculated with 100 $\mu$l of the suspension. The resulting suspension was diluted to 1 cell per ml, 32 wells were inoculated. A further dilution of 0.5 cells per ml was prepared and 24 wells were inoculated. After five days 100 $\mu$l of RPMI medium containing 15% FCS were added to each well. For example, in case of clone HG2-25 the hybridoma clones appeared in 2 of 24 wells (0.5 cells/ml) and 4 of 32 wells (1 cell/ml) after a cultivation of 10 days. Finally, the supernatant of each clone was examined for antibodies as described in (1). In this assay antibody-producing ability was observed in all clones.

### (3) Production of a Monoclonal Anti-Human IgG Antibody

A suspension of $5 \times 10^6$ cells of the identified anti-human IgG antibody-producing hybridoma in 0.5 ml physiological saline was injected into the abdominal cavity of BALB/C mice previously treated with 0.5 ml pristane. Three weeks later abdominal distension of the mice was confirmed and the ascites suspension collected. The cells were removed by centrifugation of this suspension.

### (4) Isolation and Purification of Monoclonal Anti-human IgG Antibodies

The ascites supernatant prepared as described in (3) was salted out with an aqueous saturated ammonium sulfate solution (45%). The precipitate was dissolved in 0.1 M phosphate buffer (pH 8.0) and dialyzed with the same buffer. 20 mg of the gamma fraction isolated by salting-out the ascites were dissolved in 2 ml of 0.1 M phosphate buffer (pH 8.0) and the solution was adsorbed on a column of protein A-Sepharose (Pharmacia AB). Contaminants were then eluted from the column with about 50 ml of 0.1 M phosphate buffer (pH 8.0). The monoclonal anti-human IgG antibody HG2-25 was then eluted with about 100 ml 0.1 M citrate buffer (pH 5.0).

### Example 2

In the following experiments it is shown that the monoclonal anti-human IgG antibody of the present invention has a high affinity to IgG and the IgG subclasses $IgG_1$, $IgG_2$, $IgG_3$ and $IgG_4$. However, it does not recognize and bind to human IgM, IgA, or sheep erythrocytes coated with human serum albumin. Predominantly it binds to the Fc fragment of IgG rather than to the F(ab') fragment. The isotype of the monoclonal antibody according to the present invention is $IgG_{2a}$. This was determined by an immune precipitation assay.

Said monoclonal anti-human IgG antibody was furthermore labeled with radioactive iodine ($^{125}I$) to determine the epitopic domain utilizing the inhibition properties of human IgG fragments. In this assay it was confirmed that the binding capacity of HG2-25 is inhibited by the Fc fragment but not by pFc. Thus, HG2-25 recognizes the $CH_2$-domain of human IgG.

(1) Characterization of the Monoclonal Antibody HG2-25

The supernatant of the medium in which the HG2-25-producing hybridoma was propagated is diluted with a phosphate buffered saline containing 10% FCS in two-fold dilution steps. 50 $\mu$l of the dilution obtained were placed in each well of U-bottom microtiter plates. To each well 50 $\mu$l of a 1% suspension of sheep erythrocytes sensitized with human IgG were added. After stirring with a plate mixer and incubation at room temperature for two hours the agglutination image was evaluated. The test was also carried out with sheep erythrocytes sensitized with the Fc and the F(ab')$_2$ fragments of human IgG, and with human myeloma protein (IgG and its subclasses, namely $HCD_1$, $IgG_2$, $HCD_3$, $IgG_3$, and $IgG_4$) and the agglutination image was evaluated. As a result, it was confirmed that said monoclonal antibody has a high affinity to IgG, the Fc fragment and $HCD_1$, $IgG_2$, $HCD_3$, and $IgG_4$. Table II shows the hemagglutination titers:

Table II

| | Agglutination titer |
|---|---|
| IgG | $2^{11}$ |
| HCD$_1$ | $>2^{12}$ |
| IgG$_2$ | $2^{8}$ |
| HCD$_3$ | $2^{10}$ |
| IgG$_3$ | $2^{8}$ |
| IgG$_4$ | $2^{12}$ |
| F(ab')$_2$ | $<2$ |
| Fc | $2^{8}$ |

The sources of each human IgG were the following:

IgG : Human IgG (Miles Co.),

HCD$_1$ : Myeloma protein which is produced in heavy chain disease, one of the subspecies of myeloma, belonging to the IgG$_1$ subclass,

IgG$_2$ : IgG$_2$ prepared by restrictive digestion of human IgG with papain followed by purification,

HCD$_3$ : Myeloma protein which is produced in H chain disease, one of the subspecies of myeloma, belonging to the IgG$_3$ subclass,

IgG$_3$ : Human IgG purified by passing through a protein A Sepharose column,

IgG$_4$ : Myeloma protein, which belongs to the IgG$_4$ subclass, purified by Sepharose G200 and DEAE cellulose columns,

6

$F(ab')_2$  :  Human IgG treated with pepsin followed by purification,

Fc  :  Human IgG treated with papain followed by purification.

(2) Labeling of the Monoclonal Antibody HG2-25 with Iodine (Iodogen Method)

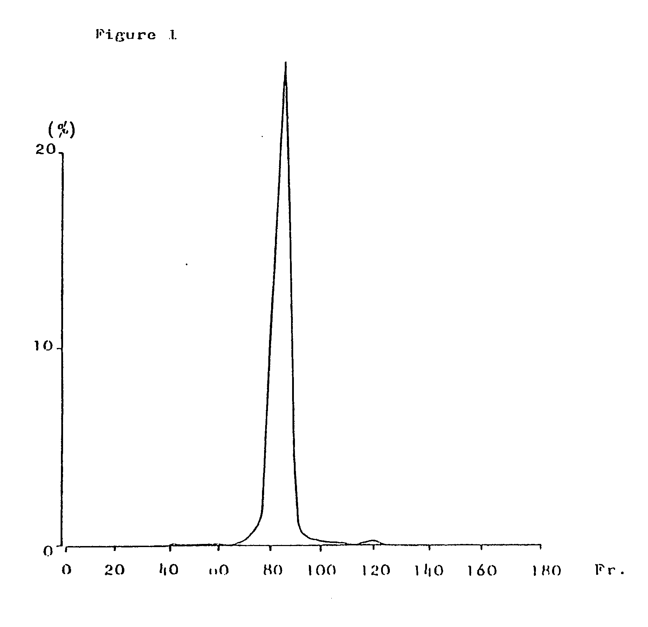

50 $\mu$l of a solution of 40 $\mu$l/ml of 1,3,4,6-tetrachloro-3$\alpha$,6$\alpha$-diphenyl glycol uryl (IODO-GEN, Pierce Chemical Co.) in dichloromethane were placed into a glass tube and dried at room temperature under nitrogen atmosphere. Then a solution of HG2-25 antibody (30 $\mu$g/15 $\mu$l) dissolved in 15 $\mu$l of 0.1 M phosphate buffer (pH 7.4) and 0.5 mCi/5 $\mu$l of radioactive sodium iodide ($Na^{125}I$) were added. The mixture was shaken moderately at room temperature for 15 minutes. The resulting reaction mixture was passed through a Sephadex G-25 (Pharmacia AB) column and eluted with a phosphate-buffered saline. The first eluted fraction contained the $^{125}I$-labeled monoclonal antibody HG2-25 and was collected. The chromatogram (Figure 1) on a Sepharose 6B column (1.6 x 56 cm) indicates that the product is pure and scarcely contains agglutinate.

(3) Recognition Site of Monoclonal Antibody HG2-25

Each well of a polystyrene microtiter plate (immulon 2 plate, Dynatech Co.) was inoculated with 100 $\mu$l of IgG solution (2 $\mu$l/ml) in 0.05 M carbonate buffer (pH 9.1). The plate was incubated overnight at 4°C. Then, the wells were washed 3 times with 0.1 M phosphate buffer solution (pH 7.4) containing 0.05 % Tween 20 and 0.01 % sodium azide and blocked with 0.1 M phosphate buffer solution containing 1 mg/ml human serum albumin. After washing with said buffer solution, solutions of human IgG, $F(ab')_2$, Fc, tImFc and pF'c (5 $\mu$g/ml, in 0.1 M phosphate buffer solution) were transferred to said plate together with $^{125}I$-labeled HG2-25 antibodies. The plate was incubated 16 hours at room temperature and then washed as described above. The labeled antibody HG2-25 specifically binding to IgG fixed to the wells was released from the IgG with 10% acetic acid. The radioactivity of the eluate was measured. As shown in Table III specific binding of the monoclonal antibody HG2-25 is inhibited up to 96% by intact human IgG. Additionally, the Fc fragment inhibits the binding of HG2-25 up to 99%. The $F(ab')_2$ and the pFc' fragments, however, scarcely inhibit the binding of HG2-25 to IgG fixed to the wells.

## Table III

| | IgG | F(ab')$_2$ | Fc | tlmFc | pFc |
|---|---|---|---|---|---|
| HG2-25 | 95% | 7% | 99% | 78% | 5% |

The sources of IgG and each fragment are the following:

IgG : human IgG (Miles Co.),

F(ab')$_2$ : human IgG treated with pepsin,

Fc : human IgG treated with papain,

tlmFc : Fc treated with thermolysin,

pFc : Fc treated with pepsin.

(4) Affinity with Human IgG in Certain Conditions

(a) Affinity with Human IgG in Solutions

The $^{125}$I-human IgG labeled by the iodogen method was dissolved in 0.1M phosphate buffer solution (pH7.4) containing 0.1% serum albumin, 0.05% Tween 20 and 0.01% sodium azide (buffer solution B) to be 0.04 $\mu$g/ml. To a mixture of 50$\mu$l of the above solution and 50$\mu$l of monoclonal anti-human IgG antibody solution (diluted with buffer solution B to 16$\mu$g/ml). 50 $\mu$l of 0.1-50 $\mu$g/ml human IgG solution were added and allowed to react in a polystyrene tube for 16 hours.

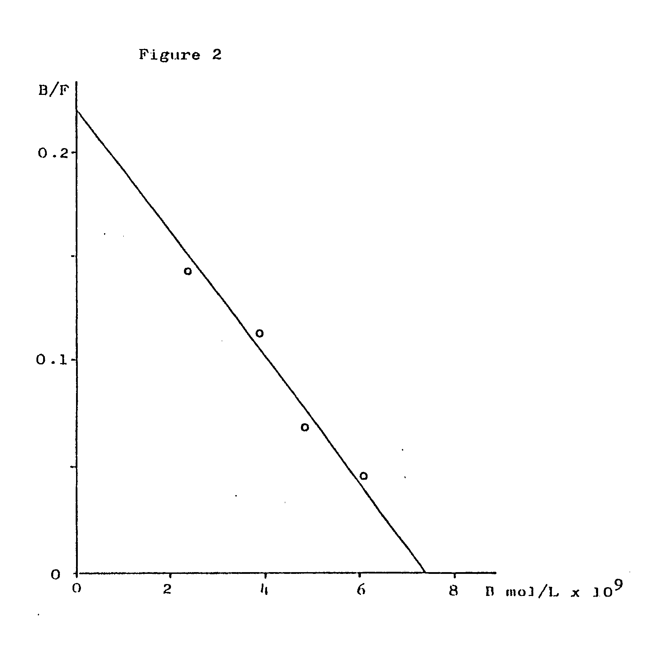

5 mg of a rabbit anti-mouse IgG antibody having a high affinity to IgG$_2$, an isotype of the monoclonal anti-human IgG antibody, were added to 1 ml of a cyanogen bromide-activated Sepharose carrier (Pharmacia AB) and allowed to bind to said carrier. The result was an immobilized anti-mouse IgG$_{2a}$ antibody. 0.1 ml of this immobilized second antibody (sufficient quantity to bind all of the monoclonal anti-human IgG antibody used) were added to each of the polystyrene tubes obtained as described above and allowed to react at room temperature for an hour while stirring. After washing 3 times with 2 ml of buffer solution B, the radioactivity was measured with a $\gamma$-counter. The affinity constant was determined according to the Scatchard method and is 2.7 x 10$^7$. Figure 2 shows the Scatchard plot.

(b) Affinity with Human IgG Specifically Binding to an Allergen Disk

50 $\mu$l of standard solution A containing 5U/ml of honey bee venom-specific IgG were added to a honey bee venom allergen disk of IgG-RAST (Phadebas IgG-RAST, Pharmacia AB) and incubated for 3 hours. 50 $\mu$l of $^{125}$I-labeled HG2-25 whose concentration was altered from 0.2 $\mu$Ci/ml to 10 $\mu$Ci/ml were added, and the reaction mixture was incubated at 25°C for 16-18 hours. After washing 3 times with the wash solution described above, the radioactivity was measured.

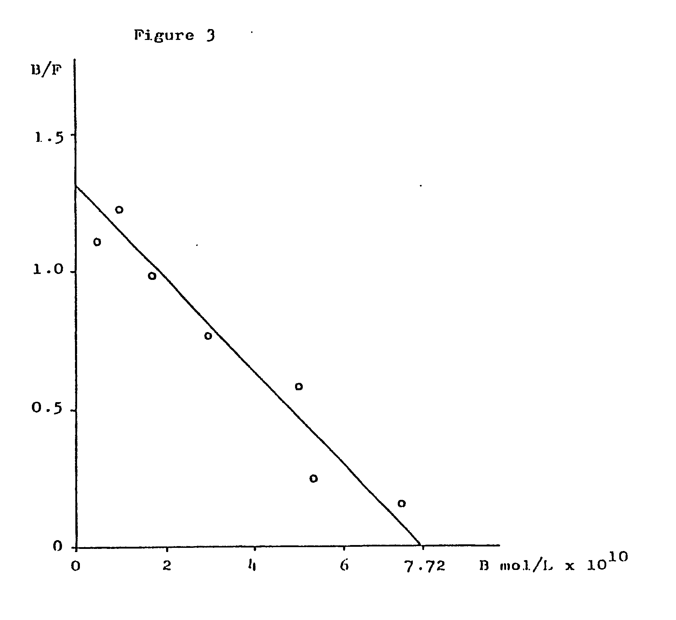

The affinity constant determined from this experiment was 3.5 x 10$^9$. Figure 3 shows the Scatchard plot.

(c) Affinity to Human IgG Nonspecifically Bound to a Microtiter Plate

50 $\mu$l of 0.25M carbonate buffer solution (pH 9.1) containing 2 $\mu$g/ml human IgG were transferred into each well of polystyrene microtiter plates. These plates were incubated at 37°C for 2 hours, washed with 0.1M phosphate buffer (pH 7.4) containing 0.1% serum albumin, 0.05% Tween 20$^R$ and 0.01% sodium azide (buffer solution A) and then incubated with buffer solution A at 37°C for an hour. Subsequently, after washing 2 times with the buffer solution A, 50 $\mu$l of $^{125}$I-labeled HG2-25 antibody solution (0.0003 - 3 $\mu$Ci/ml) were added to each well. After incubating 16 hours at room temperature these plates were washed two times with buffer solution A. The $^{125}$I-labeled HG2-25 antibody binding to human IgG on the plates was removed from the plates with 10% acetic acid and transferred to tubes for measurement. Then the radioactivity was measured. The affinity constant (K) was calculated from the result of the measurements according to the Scatchard method. The calculated value is K = 1.5 x 10$^9$M$^{-1}$.

## Claims

1. Monoclonal anti-human IgG antibodies displaying the following features:
   a) recognizing the CH$_2$- domain of human IgG,
   b) exhibiting high affinity to human IgG and the IgG subclasses IgG$_1$, IgG$_2$, IgG$_3$, and IgG$_4$,
   c) binding more preferentially to the allergen-specific IgG than to the non-specific IgG, and
   d) exhibiting substantially no affinity to free human IgG in the form of a solution.

2. Process for preparing the monoclonal antibodies according to claim 1 comprising the following steps:
   Fusion of myeloma cells and antibody-producing cells of an animal immunized with human IgG, selecting from the resulting hybridomas an anti-human IgG antibody-producing hybridoma, cloning said hybridoma by limiting dilution to obtain clones which produce monoclonal antibodies having high affinities to human IgG, multiplying said clone and recovering the monoclonal antibodies produced by said clone which bind equally to IgG$_1$, IgG$_2$, IgG$_3$ and IgG$_4$ and preferentially to allergen-binding human IgG , but exhibit substantially no affinity to free human IgG in the form of a solution.

3. Process according to claim 2 comprising the following steps:
   Injection of the clone into the abdominal cavity of a mouse previously treated with pristane where said clone is propagated and produces the monoclonal antibodies according to claim 1,collecting the ascites containing the monoclonal antibodies and purification of the monoclonal antibodies.

4. Process according to claim 2 wherein the crude monoclonal antibodies are purified by affinity chromatography using a protein A-binding carrier resin.

5. The process of claim 4 wherein said carrier resin is Sepharose®.

## Revendications

1. Anticorps monoclonaux anti-IgG humaines présentant les caractéristiques suivantes de :
   a) reconnaître le domaine CH$_2$ des IgG humaines ;
   b) présenter une affinité élevée pour les IgG humaines et les sous-classes d'IgG = IgG$_1$, IgG$_2$, IgG$_3$ et IgG$_4$ ;
   c) se lier de façon davantage préférée aux IgG spécifiques d'un allergène plutôt qu'aux IgG non-spécifiques; et
   d) ne manifester pratiquement pas d'affinité pour les IgG humaines libres sous la forme d'une solution.

2. Procédé de préparation des anticorps monoclonaux selon la revendication 1, comprenant les étapes suivantes :
   - fusion de cellules de myélome et de cellules productrices d'anticorps d'un animal immunisé par des IgG humaines ;
   - sélection parmi les hybridomes résultants d'un hybridome producteur d'anticorps anti-IgG humaines ;
   - clonage dudit hybridome par dilution limitante pour obtenir des clones qui produisent des anticorps monoclonaux ayant des affinités élevées pour les IgG humaines ;
   - multiplication dudit clone ; et
   - récupération des anticorps monoclonaux produits par ledit clone qui se lient de façon égale aux IgG$_1$, IgG$_2$, IgG$_3$ et IgG$_4$ et, préférentiellement, aux IgG humaines se liant à un allergène, mais ne

EP 0 163 141 B1

présentent pratiquement pas d'affinité pour les IgG humaines libres sous la forme d'une solution.

3. Procédé selon la revendication 2, comprenant les étapes suivantes :
- injection du clone dans la cavité abdominale d'une souris préalablement traitée par du pristane, cavité dans laquelle ledit clone se propage et produit les anticorps monoclonaux selon la revendication 1 ;
- collecte des ascites contenant les anticorps monoclonaux ; et
- purification des anticorps monoclonaux.

4. Procédé selon la revendication 2, dans lequel les anticorps monoclonaux bruts sont purifiés par chromatographie d'affinité utilisant une résine support se liant à la protéine A.

5. Procédé selon la revendication 4, dans lequel ladite résine support est le Sepharose®.

**Patentansprüche**

1. Monoclonale anti-human-IgG-Antikörper mit den folgenden Merkmalen:
(a) sie erkennen die $CH_2$-Domäne von human-IgG,
(b) sie haben eine hohe Affinität zu human-IgG und den IgG-Subklassen $IgG_1$, $IgG_2$, $IgG_3$ und $IgG_4$,
(c) sie binden bevorzugter an Allergen-spezifisches IgG als an nicht-spezifisches IgG, und
(d) sie haben keine wesentliche Affinität zu freiem human-IgG in Form einer Lösung.

2. Verfahren zur Herstellung der monoclonalen Antikörper nach Anspruch 1, umfassend die folgenden Schritte:
Fusion von Myelomzellen und Antikörper-bildenden Zellen eines mit human-IgG immunisierten Tieres, Selektion eines anti-human-IgG-Antikörper-bildenden Hybridoms aus den erhaltenen Hybridomen, Clonierung des Hybridoms bei Verdünnung unter limitierenden Bedingungen, um Clone zu erhalten, die monoclonale Antikörper mit hohen Affinitäten zu human-IgG bilden, Vermehrung der Clone und Gewinnung von durch die Clone gebildeten monoclonalen Antikörpern, die gleichermaßen an $IgG_1$, $IgG_2$, $IgG_3$ und $IgG_4$ und bevorzugt an Allergen-bindendes human-IgG binden, aber keine wesentliche Affinität zu freiem human-IgG in Form einer Lösung haben.

3. Verfahren nach Anspruch 2, umfassend die folgenden Schritte:
Injektion des Clons in die Abdominalhöhle einer Maus, die vorher mit Pristan behandelt wurde, wo der Clon vermehrt wird und die monoclonalen Antikörper nach Anspruch 1 bildet, Sammeln des Ascites enthaltend die monoclonalen Antikörper und Reinigung der monoclonalen Antikörper.

4. Verfahren nach Anspruch 2, wobei die rohen monoclonalen Antikörper durch Affinitätschromatographie mit einem Protein A-bindenden Trägerharz gereinigt werden.

5. Verfahren nach Anspruch 4, wobei das Trägerharz Sepharose® ist.

10

Figure 1

Figure 2

B/F (vertical axis)

0.2

0.1

0

0    2    4    6    8    B mol/L x 10$^9$

Figure 3